# EUROPEAN PATENT APPLICATION

(11) **EP 1 772 057 A2**
(43) Date of publication of application: **11.04.2007**
(21) Application number: 06255042.1
(22) Date of filing: 29.09.2006
(51) Int. Cl.: A01N 59/20, A01N 59/16, A01P 1/00, A01N 25/22

(54) **Method for disinfecting or sanitizing a surface**

(30) Priority: 07.10.2005 US 725062 P
(71) Applicant: ROHM AND HAAS COMPANY, Philadelphia, Pennsylvania 19106-2399 (US)
(72) Inventor: Chia, Li-Lang, Ambler Pennsylvania 19002 (US); Ghosh, Tirthankar, Oreland Pennsylvania 19075 (US); Weinstein, Barry, Dresher Pennsylvania 19025 (US); Williams, Terry Michael, Lower Gwynedd Pennsylvania 19002 (US)
(74) Representative: Kent, Venetia Katherine

(57) **Abstract**

A method for disinfecting or sanitizing a surface by applying an antimicrobial composition comprising a metal-polymer complex.

## Description

The present invention relates to a method for disinfecting or sanitizing a surface by applying an antibacterial composition.

The constant threat of bacterial contamination and the associated repercussions on health have made antimicrobial solutions a ubiquitous part of commercial and residential cleaning and disinfection processes. Dilute aqueous detergents show no detectable reduction in bacterial levels on surfaces amenable to bacterial growth and proliferation in susceptible environments, such as hospitals and in residential kitchen and bath areas. On the other hand, oxidants such as aqueous hypochlorite and phenolic compositions produce substantial reductions in bacterial levels that are relatively short-lived (3 to 6 hours). This often results in recontamination due to reuse of such surfaces, requiring frequent reapplication of disinfectant. Further, relatively high concentrations of the active agent have to be incorporated in such formulations to obtain broad spectrum disinfection. These high concentrations often have undesirable side effects such as skin and eye irritation, in addition to being potentially hazardous when in contact with food. There is therefore a need for the development of new disinfecting formulations that can provide sustained broad spectrum microbial disinfection on surfaces over prolonged periods without reapplication, even after being contacted by cleaning solutions and after surface reuse. Furthermore, it is desirable to achieve disinfecting action without toxicity problems for the user.

A number of microbicides capable of exhibiting antibacterial activity when contained in coating compositions, resin moldings, papers and binders have been proposed. Among them are inorganic microbicides, i.e., inorganic compounds on which metal ions are supported. Examples of inorganic microbicides include metals on active carbon, apatite, zeolite, and various phosphates.

Compositions containing the inorganic microbicides frequently discolor upon exposure to heat and/or light. One method for inhibiting such discoloration is provided by Ohsumi et al. in U.S. Patent No. 5,698,229. Ohsumi et al. disclose the combination of an inorganic compound on which silver ions are supported with a compound of the following formula: 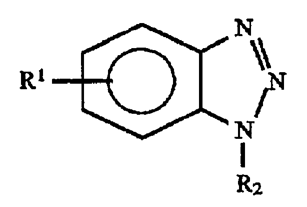 wherein R¹ is hydrogen or a lower alkyl group and R² is hydrogen or an alkali metal.

Nevertheless, there remains a need for new disinfecting methods using compositions which exhibit the positive antibacterial activity of metal ions without the undesirable heat and light stability problems often associated with compositions incorporating such metal ions.

### STATEMENT OF THE INVENTION

In a first aspect of the present invention, there is provided a method for sanitizing a surface; said method comprising applying to the surface an antimicrobial composition comprising: (a) at least 50% of an alcohol selected from ethanol and isopropanol; and (b) a metal complexed with a polymer, wherein the metal is selected from copper, silver, gold, tin, zinc and combinations thereof, alternatively the metal is selected from copper, silver, gold and combinations thereof, alternatively the metal is selected from copper, silver and combinations thereof, alternatively the metal is silver; and, wherein the polymer comprises monomer residues selected from residue A, residue B, residue C and mixtures thereof, with the proviso that the polymer contains no more than 99.5 wt% of monomer residues of residue B, alternatively no more than 99 wt% of monomer residues of residue B, alternatively no more than 98 wt% monomer residues of residue B, alternatively no more than 95 wt% of monomer residues of residue B, alternatively no more than 90 wt% of monomer residues of residue B;
wherein residue A is 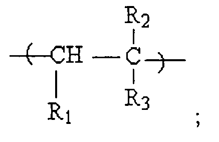 wherein residue B is 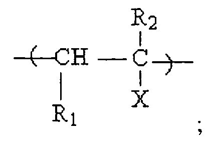 and
wherein residue C is 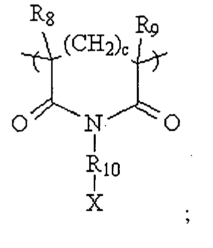 wherein
X is selected from an unsaturated or aromatic heterocycle having at least one hetero atom selected from N, O and S; alternatively X is selected from an unsaturated or aromatic heterocycle having at least one hetero N atom;
c is 0 or 1; alternatively c is 0;
R₁ is selected from H, CH₃ and -CO₂R₄; where R₄ is selected from H, CH₃, C₂H₅, a C₃-C₂₄ alkyl;
R₂ is selected from H, CH₃, C₂H₅, phenyl, -CH₂CO₂R₅ and -CO₂R₅; where R₅ is selected from (I)-(V),
(I) H;
(II) 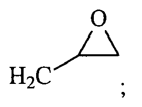
(III) -(CH₂CH(R₁₁)O)ₙH;
(IV) -(CH₂CH(R₁₁)O)ₙCOCH₂COCH₃; and,
(V) 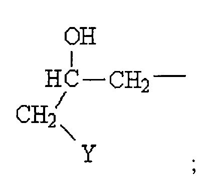
where R₁₁ is selected from H, methyl and phenyl; n is an integer from 1 to 20; Y is selected from OH, SO₃Z and X; where Z is selected from H, sodium, potassium and NH₄⁺; with the proviso that when the polymer contains 0 wt% of monomer residues of residue B and 0 wt% of monomer residues of residue C, R₂ is -CH₂CO₂R₅ or -CO₂R₅, R₅ is (V) and Y is X;
R₃ is selected from H, methyl, phenyl, sulfonated phenyl, phenol, acetate, hydroxy, a fragment O-R₁, where R₁ is as defined previously, -CO₂R₁₂ and -CONR₆R₇; where R₆ and R₇ are independently selected from H, methyl, ethyl, C(CH₃)₂CH₂SO₃Z, where Z is as defined previously, C₃-C₈ alkyl and a combined ring structure and R₁₂ is selected from H, CH₃, C₂H₅ and C₃-C₂₄ alkyl;
R₈ and R₉ are independently selected from hydrogen, methyl, ethyl and C₃-C₄ branched or straight chain alkyl; alternatively R₈ and R₉ are both hydrogen;
R₁₀ is selected from C₁-C₈ alkyl, C₂-C₈ alkenyl, C₆-C₁₀ unsaturated acyclic, C₆-C₁₀ cyclic, C₆-C₁₀ aromatic, C₂-C₄ alkylene oxide and poly (C₂-C₄ alkylene)_{b} oxides; where b is an integer from 2 to 20; alternatively R₁₀ is selected from C₂-C₈ branched and straight chain alkyl groups.

In another aspect of the present invention, the antimicrobial composition comprises: (a) at least 50% of an alcohol selected from ethanol and isopropanol; and (b) a metal complexed with a polymer, wherein the metal is selected from copper, silver, gold, tin, zinc and combinations thereof, alternatively the metal is selected from copper, silver, gold and combinations thereof, alternatively the metal is selected from copper, silver and combinations thereof, alternatively the metal is silver; and, wherein the polymer comprises at least 0.5 wt% crosslinker and at least 5 wt%, alternatively at least 75 wt%, alternatively at least 80 wt%, alternatively at least 85 wt%, alternatively at least 90 wt%, alternatively at least 95 wt% of monomer residues selected from residue A, residue B, residue C and mixtures thereof; wherein residue A, residue B and residue C are as previously defined.

### DETAILED DESCRIPTION OF THE INVENTION

All percentages stated herein are by weight, unless specified otherwise. As used herein and in the appended claims, the term "silver" refers to silver metal that is incorporated into an antimicrobial composition of the present invention. While not wanting to be bound as to the oxidation state of the silver (Ag⁰, Ag¹⁺ or Ag²⁺), that is incorporated into the antimicrobial composition, silver may be added to the antimicrobial composition by washing the polymer in a silver solution such as silver nitrate in deionized water ("DI"). Aside from DI, other liquid mediums can also be used such as water, aqueous buffered solutions and organic solutions such as polyethers or alcohols. Other sources of silver include but are not limited to silver acetate, silver citrate, silver iodide, silver lactate, silver picrate and silver sulfate. The concentration of silver in these solutions can vary from the concentration required to add a known quantity of silver to the antimicrobial composition to a saturated silver solution.

In another embodiment of the present invention, the antimicrobial composition contains at least 10 ppm of the metal; alternatively at least 25 ppm of the metal; alternatively at least 50 ppm metal; alternatively at least 75 ppm metal; the antimicrobial composition contains no more than 500 ppm metal; alternatively no more than 300 ppm metal; alternatively no more than 200 ppm metal; alternatively no more than 150 ppm metal. In one embodiment of the present invention, the metal comprises silver.

The term "alkyl" as used herein and in the appended claims includes both straight chain, branched and cyclic alkyl groups.

The term "alkenyl" as used herein and in the appended claims includes both straight chain and branched chain alkenyl groups.

Unsaturated or aromatic heterocycles suitable for use with the present invention include, for example, 5 to 7-membered heterocycles having some degree of unsaturation; aromatic heterocycles having at least one hetero atom selected from N, O and S atoms; isomers of such heterocycles and combinations thereof. In addition, suitable heterocycles may include, for example, 5 to 7-membered heterocycles that are fused together to form larger 9 to 14 membered heterocycles having at least one N, O or S atom; isomers of such heterocycles and combinations thereof. Additional heterocycles suitable for use with the present invention include 5 to 7-membered heterocycles that are fused with a carbocycle to form larger 9 to 14-membered heterocycles.

In another embodiment, the antimicrobial compositions of the present invention include a polymer comprising a heterocyclic group selected from imidazole; thiophene; pyrrole; oxazole; thiazoles and their respective isomers (e.g., thiazol-4-yl, thiazol-3-yl and thiazol-2-yl); tetrazole; pyridine; pyridazine; pyrimidine; pyrazine; azoles; indazoles; triazoles and their respective isomers (e.g., 1,2,3-triazole and 1,2,4-triazole); and combinations thereof, such as imidazole 1,2,3-triazole-1,2,4-triazole; benzotriazole; methyl-benzotriazole; benzothiazole; methylbenzothiazole; benzimidazole and methyl benzimidazole. In one aspect of this embodiment, the antimicrobial compositions of the present invention include a polymer comprising a heterocycle group selected from imidazole, benzotriazole and benzimidazole.

In another embodiment of the present invention, the antimicrobial composition comprises a heterocyclic containing monomer and a non-heterocyclic containing monomer. In one aspect of this embodiment, the ratio of the heterocyclic containing monomer to the non-heterocyclic containing monomer is 95:5 to 5:95; alternatively 80:20 to 20:80; alternatively 60:40 to 40:60. In one aspect of this embodiment, the heterocyclic containing monomer is vinylimidazole.

In another embodiment of the present invention, the antimicrobial composition comprises a heterocyclic containing monomer complexed with silver. In one aspect of this embodiment, the weight ratio of the heterocyclic containing monomer to silver is 95:5 to 5:95; alternatively 90: 10 to 10:90; alternatively 80:20 to 20:80. In one aspect of this embodiment, the molar ratio of the silver to the heterocyclic containing monomer is 10:1 to 1:10; alternatively 4:1 to 1:4; alternatively 2:1 to 1:2. In one aspect of this embodiment, the heterocyclic containing monomer is vinylimidazole. In another aspect of the invention, the polymer is a copolymer of 1-vinylimidazole, (meth)acrylic acid(s), and alkyl acrylate(s); alternatively C₄-C₁₂ alkyl acrylates and acrylic acid. In this aspect, preferably 1-vinylimidazole is present in an amount from 35-50%, (meth)acrylic acid is present in an amount from 5-15% and alkyl acrylate is present in an amount from 35-50%. In this aspect, the copolymer also may contain cross-linker(s).

In another embodiment of the present invention, the polymer comprises 0.5 to 60 wt% cross-linker, alternatively at least 2 wt% cross-linker, alternatively at least 5 wt% cross-linker, alternatively at least 8 wt% cross-linker, alternatively at least 10 wt% cross-linker; alternatively no more than 40% cross-linker, alternatively no more than 30% cross-linker, alternatively no more than 20% cross-linker, alternatively no more than 15% cross-linker. In another embodiment of the invention, the polymer is made with less than 0.5% cross-linker, or alternatively is made substantially without cross-linker.

Cross-linkers suitable for use with the present invention include any known cross-linking material provided that the physical and chemical stability of the antimicrobial composition is substantially unaffected by inclusion of the cross-linking material. Examples of cross-linkers suitable for use with the present invention included, but are by no means limited to, di-, tri-, tetra- and higher multi-functional ethylenically unsaturated monomers such as, trivinylbenzene; divinyltoluene; divinylpyridine; divinylnaphthalene; divinylxylene; ethyleneglycol diacrylate; trimethylolpropane triacrylate; diethyleneglycol divinyl ether; trivinylcyclohexane; allyl methacrylate ("ALMA"); ethyleneglycol dimethacrylate ("EGDMA"); diethyleneglycol dimethacrylate ("DEGDMA"); propyleneglycol dimethacrylate; propyleneglycol diacrylate; trimethylolpropane trimethacrylate ("TMPTMA"); divinylbenzene ("DVB"); 2,2-dimethylpropane-1,3-diacrylate; 1,3-butyleneglycol diacrylate; 1,3-butyleneglycol dimethacrylate; 1,4-butanediol diacrylate; diethyleneglycol diacrylate; diethyleneglycol dimethacrylate; 1,6-hexanediol diacrylate; 1,6-hexanediol dimethacrylate; tripropyleneglycol diacrylate; triethyleneglycol dimethacrylate; tetraethyleneglycol diacrylate; polyethyleneglycol 200 diacrylate; tetraethyleneglycol dimethacrylate; polyethyleneglycol dimethacrylate; ethoxylated bisphenol A diacrylate; ethoxylated bisphenol A dimethacrylate; polyethyleneglycol 600 dimethacrylate; poly(butanediol) diacrylate; pentaerythritol triacrylate; trimethylolpropane triethoxy triacrylate; glycerylpropoxy triacrylate; pentaerythritol tetraacrylate; pentaerythritol tetramethacrylate; dipentaerythritol monohydroxypentaacrylate; divinyl silane; trivinyl silane; dimethyl divinyl silane; divinyl methyl silane; methyl trivinyl silane; diphenyl divinyl silane; divinyl phenyl silane; trivinyl phenyl silane; divinyl methyl phenyl silane; tetravinyl silane; dimethyl vinyl disiloxane; poly(methyl vinyl siloxane); poly(vinyl hydrosiloxane); poly (phenyl vinyl siloxane) and mixtures thereof.

In another embodiment of the present invention, the antimicrobial compositions comprise a polymer made with a cross-linker selected from allyl methacrylate (ALMA); ethyleneglycol dimethacrylate (EGDMA); diethyleneglycol dimethacrylate (DEGDMA); trimethylolpropane trimethacrylate (TMPTMA) and divinylbenzene (DVB). In one aspect of this embodiment, the antimicrobial compositions comprise a polymer made with trimethylolpropane trimethacrylate (TMPTMA).

In another embodiment of the present invention, the polymer, of which the antimicrobial composition is comprised, exhibits an average particle size of less than 200 nm; alternatively less than 50 nm; alternatively of 1 to 10 nm; alternatively less than 10 nm; alternatively of 1 to 8 nm; alternatively of less than 5 nm. In another embodiment, in which the polymer is made substantially without cross-linker, the polymer does not have a definable particle size.

In another embodiment of the present invention, the polymer, of which the antimicrobial composition is comprised, exhibits a molecular weight of less than 500,000; alternatively of less than 100,000; alternatively of less than 50,000; alternatively of 500 to 5,000.

The antimicrobial composition of the present invention comprises at least 50% of a solvent selected from ethanol and isopropanol. In one preferred embodiment, the solvent is ethanol. Preferably, water is also present in the composition. In one embodiment of the invention, the amount of ethanol or isopropanol is at least 55%, alternatively at least 58%; alternatively no more than 80%, alternatively no more than 75%, alternatively no more than 70%. In one embodiment of the invention, the composition is substantially free of solvents other than ethanol, isopropanol and water. Other solvents and additives that may be present in antimicrobial compositions used in hand sanitizers, gels and wipes include glycols, especially propylene glycol; glycerine; esters, especially isopropyl myristate; aminomethylpropanol; Carbomer^{™} polymers, or other polymers added for rheology control; fragrances; natural products; amines; chelants; pH buffers; etc.

In another embodiment of the present invention, the antimicrobial composition is light stable. In one aspect of this embodiment, upon prolonged exposure of an antimicrobial system of the present invention to light in the visible spectrum, the individual values of Hunter L, a, b and L*a*b* (CIELAB) for the antimicrobial system exhibit a change from such exposure of less than a factor of 3; alternatively of less than a factor of 2. For a description of the Hunter Color test methods, see Billmeyer, Jr. et al., PRINCIPLES OF COLOR TECHNOLOGY, John Wiley & Sons, 2ED (1981).

The term "prolonged exposure" as used herein and in the appended claims means an intermittent exposure period of at least 24 hours; alternatively an intermittent exposure period of at least one week; alternatively an intermittent exposure period of at least one year; alternatively an intermittent exposure period of at least two years; alternatively an intermittent exposure period of at least five years. The term "intermittent exposure period" as used herein and in the appended claims refers to a period during which the exposure to light in the visible spectrum is not constant. An example of an intermittent exposure period of 24 hours would be an ambient, outdoor light cycle from dawn to dawn.

The antimicrobial composition used in the present invention inhibits the adhesion of bacteria or other microbes to a surface, inhibits the growth of bacteria or other microbes on the surface, and kills bacteria or other microbes on the surface or in a radius extending from a point of application. The antimicrobial composition of the present invention inhibits microbial production by at least 25%; alternatively, the antimicrobial composition of the present invention exhibits at least a 1-log reduction (≥90% inhibition) of microbial colony forming units per mL; alternatively the antimicrobial composition of the present invention exhibits at least a 2-log reduction (≥99% inhibition) of microbial colony forming units per mL; alternatively the antimicrobial composition of the present invention exhibits at least a 3-log reduction (≥99.9% inhibition) of microbial colony forming units per mL - this level of reduction is referred to herein as "sanitization"; alternatively the antimicrobial composition of the present invention exhibits at least a 6-log reduction (≥99.9% inhibition) of microbial colony forming units per mL - this level of reduction is referred to herein as "disinfection." Such microbes include, but are not limited to, *Aureobasidium pullulans, Bacillus cereus, Bacillus thuringiensis, Chaetomium globosum, Enterobacter aerogines, Escherichia coli, Gliocladtum virens, Klebsiella Pheumoniae, Legionella pneumpophila, Listeria Monocytogenes, Mycobacterium tuberculosis, Porphyromonas gingivalis, Proteus mirabilis, Proteus vulgaris, Pseudomonas aeruginosa, Saccharomyces cerevisiae, Salmonella gallinarum, Salmonella typhimurium, Staphylococcus aureus, Staphylococcus epidermidis, Streptococcus agalactiae, Streptococcus faecalis, Streptococcus mutans, Trycophyton malmsten, Vibrio parahaemolyticus, Stachybotrys, Aspergillus niger, Candida albicans and Penicillium funiculosum.*

The antimicrobial compositions of the present invention are deposited on the surface of a substrate to form an antimicrobial layer thereon. The surface may be a hard surface, i.e., one which is non-porous, such as a countertop, cabinet, doorknob, fixture, etc., and constructed from glass, ceramic, stone, metal, plastic, finished wood or composite materials, including, e.g., fiberglass and other plastic/glass and plastic/ceramic composites. Alternatively, the surface may be porous surfaces such as clay, wood, leather, fabric, rubber, paper and skin.

In one embodiment, the antimicrobial composition of the present invention may be used as a disinfectant spray. Pump and aerosol sprays are suitable. Other methods of application include wiping and applying the composition in the form of a gel. A typical application by means of spraying could entail spraying for 1-10 seconds at a distance of 2-12 inches from the surface to provide complete coverage and saturation of the treated area. Such an application would provide from 0.01 to 0.5 g/cm² of material on the surface. For optimal results, the surface should remain wet for at least 30 seconds. A typical application by means of wiping could entail applying the composition with a textile or non-woven fabric to thoroughly wet the surface such that it remains wet for at least 30 seconds. Such an application would provide from 0.01 to 0.5 g/cm² of material on the surface. A typical application in the form of a gel could entail pumping gel from a pump bottle to cover the surface, which should remain wet for at least 30 seconds. Such an application would provide from 0.1 to 1 g/cm² of material on the surface.

In another embodiment, the antimicrobial compositions of the present invention may optionally include one or more antimicrobial agents, provided that the physical and chemical stability of the antimicrobial composition is substantially unaffected by such inclusion. Antimicrobial agents suitable for use with the present invention include, for example, 3-isothiazolones; 3-iodo-2-propynylbutylcarbamate; 2-bromo-2-nitropropanediol; glutaric dialdehyde; 2-n-octyl-3-isothiazolone; sodium 2-pyridinethiol-1-oxide; p-hydroxy benzoic acid alkyl ester; tris(hydroxymethyl)nitromethane; dimethylol-dimethyl-hydantion; benzisothiazolone; and 2,4,4'-trichloro-2'-hydroxy-diphenyl ether.

In another embodiment, the antimicrobial compositions of the present invention may optionally include one or more disinfecting agents, provided that the physical and chemical stability of the antimicrobial composition is substantially unaffected by such inclusion. Disinfecting agents suitable for use with the present invention include, for example, quaternary ammonium disinfectants and phenolic disinfectants.

Some embodiments of the present invention will now be described in detail in the following Examples. All fractions and percentages set forth below in the Examples are by weight unless otherwise specified.

### Examples 1-4

### Preparation of polymer product

Polymer products were prepared using the following process:
(a) isopropanol (515 g of 99 wt%) was charged to a one liter kettle equipped with a stirrer, dropping funnel and a condenser;
(b) the contents of the kettle where heated to 80 °C with constant gentle agitation;
(c) for each of Examples 1-4, a mixture with the composition set forth in Table 1 was slowly added to the kettle dropwise over a two hour period, while maintaining the temperature of the kettle contents at 80°C with constant gentle agitation;
(d) the product of (c) was maintained at 80°C with constant gentle agitation for a period of thirty minutes;
(e) t-amyl peroxypivalate (2 g) in isopropanol (5 g of 99 wt%) was added to the product of (d);
(f) the product of (e) was maintained at 80°C with constant gentle agitation for a period of thirty minutes;
(g) t-amyl peroxypivalate (2 g) in isopropanol (5 g of 99 wt%) was added to the product of (f);
(h) the product of (g) was maintained at 80°C with constant gentle agitation for a period of thirty minutes;
(i) t-amyl peroxypivalate (2 g) in isopropanol (5 g of 99 wt%) was added to the product of (h);
(j) the product of (i) was maintained at 80°C with constant gentle agitation for a period of thirty minutes;
(k) the heating source was removed and the product of (j) was allowed to cool to room temperature; and,
(l) the isopropanol was removed from the product of (k) under vacuum to leave the polymer product.

**Table I**

| Component | Example 1 Mixture Composition | Example 2 Mixture Composition | Example 3 Mixture Composition | Example 4 Mixture Composition |
|---|---|---|---|---|
| butyl acrylate (BA) | 40 g | 40 g | 45 g | 40 g |
| 1-vinylimidazole (VI) | 40 g | 50 g | 45 g | 0 g |
| 1-vinylpyrrolidone | 0 g | 0 g | 0 g | 40 g |
| acrylic acid (AA) | 10 g | 0 g | 10 g | 10 g |
| trimethylolpropane triacylate (TMPTA) | 10 g | 10 g | 0 g | 10 g |
| t-amyl peroxypivalate | 2 g | 2 g | 2 g | 2 g |
| isopropanol | 25 g | 25 g | 25 g | 25 g |

### Example 5

### Preparation of silver complex with crosslinked, imidazole containing polymer

A silver complex was prepared as follows:
(a) a uniform sample of the polymer product from Example 1 (3 g) was dispersed in deionized water (17 g);
(b) ethanol (17 g of 95 wt%) was added to product of (a) with agitation;
(c) an aqueous solution of silver nitrate (0.44 g AgNO₃ in 5 g of deionized water) was added to product of (b) with agitation, forming a white precipitate;
(d) an aqueous ammonium hydroxide solution (4.4 g of a 5 wt% solution) was added to the product of (c) with agitation forming a product clear light yellow colored solution containing 0.53 wt% silver.

### Example 6

### Preparation of control

A non-silver containing complex was prepared as follows:
(a) a uniform sample of the polymer product from Example 1 (9 g) was dispersed in deionized water (51 g);
(b) ethanol (51 g of 95 wt%) was added to the product of (a) with agitation;
(c) an aqueous ammonium hydroxide solution (12.3 g of a 5wt% solution) was added to the product of (b) with agitation forming a product non-silver containing complex.

### Example 7

### Preparation of silver complex with imidazole containing polymer

A silver complex was prepared as follows:
(a) a uniform sample of the polymer product from Example 3 (15 g) was mixed with deionized water (85 g) and an aqueous ammonium hydroxide solution (15 g of a 10wt%);
(b) an aqueous silver nitrate solution (2.2 g AgNO₃ in 10 g or deionized water) was added to the product of (a) with agitation, forming a hazy light yellow colored solution;
(c) the product of (b) was filtered, leaving a product clear light yellow filtrate containing 0.62 wt% silver.

### Example 8

### Preparation of silver complex with pyrrolidone containing polymer

A silver complex was prepared as follows:
(a) a uniform sample of the polymer product from Example 4 (16.5 g) was mixed with deionized water (6.2 g);
(b) isopropanol (6 g) and an aqueous ammonium hydroxide solution (15 g of 10 wt% solution) was added to the product of (a) with agitation;
(b) an aqueous silver nitrate solution (2.2 g AgNO₃ in 10 g deionized water) was added to the product of (b) with agitation, forming a product colorless clear solution containing 0.63 wt% silver.

### Example 9

### Preparation of silver complex with crosslinked, imidazole containing polymer (without ammonia)

A silver complex was prepared as follows:
(a) a uniform sample of the polymer product from Example 1(3.7 g) was dispersed in deionized water (6.2 g);
(b) isopropanol (6 g of 99 wt%) and 2-amino-2-methylpropanol (1.5 g) were added to the product of (a) with agitation;
(c) an aqueous silver nitrate solution (0.7 g AgNO₃ in 2 g of deionized water) was added to product of (b) with agitation, forming a product light yellow solution containing 2.2 wt% silver.

### Example 10

### Preparation of silver complex with crosslinked, imidazole containing polymer (with ammonia)

A silver complex was prepared as follows:
(a) a uniform sample of the polymer product from Example 1 (3 g) was dispersed in deionized water (17 g);
(b) ethanol (20 g of 95 wt%) was added to the product of (a) with agitation;
(c) an aqueous silver nitrate solution (0.2 g AgNO₃ in 2 g of deionized water) was added to the product of (b) with agitation, forming a gummy white precipitate;
(d) an aqueous ammonium hydroxide solution (1.7 g of a 14 wt% solution) was added to the product of (c) with agitation, forming a product clear light yellow colored solution containing 0.31 wt% silver.

### Example 11

### Preparation of silver complex with crosslinked, imidazole and polyvinylpyrrolidone containing polymer

A silver complex was prepared as follows:
(a) a uniform sample of the polymer product from Example 1 (3 g) was dispersed in deionized water (17 g);
(b) ethanol (20 g of 95 wt%) was added to the product of (a) with agitation;
(c) an aqueous silver nitrate solution (0.2 g AgNO₃ in 2 g of deionized water) was added to the product of (b) with agitation, forming a white precipitate;
(d) polyvinylpyrrolidone (0.4 g) was added to the product of (c) with agitation, forming a product clear light yellow colored solution containing 0.32 wt% silver.

### Example 12

### Stability of films formed using products of Examples 5 and 8

The product of Example 5 was drawn on a glass slide to form a film. The product of Example 8 was similarly drawn on a separate glass slide forming clear and colorless films. The films were allowed to dry on the glass slides at room temperature overnight. The next day the glass slides with their clear and colorless films were placed on a window sill that was exposed to natural sunlight for a period of sixty (60) days. At the end of the sixty (60) day period, the film made from the product of Example 5 remained clear and colorless. The film made from the product of Example 8, however, exhibited a dark reddish black appearance.

### Example 13

### Disinfection Efficacy of Silver Containing Films

*Staphylococcus aureus* of ATCC 6538 strain was grown in a growth media (Nutrient Broth) and incubated at 37 °C. Two sets of microscope cover glasses were inoculated with 10 µl of inoculum containing about 1x10⁶ bacteria per square inch of microscope cover glass. The microscope cover glasses were then dried at 37 °C for 30 to 40 minutes. One set of microscope cover glasses was then treated by spraying thereon a sample of the product solution of Example 10 diluted to 90 ppm silver. The other set of microscope cover glasses was then treated by spraying thereon a sample of the product solution of Example 11 diluted to 90 ppm silver. Survivors were recovered by placing the microscope cover glasses in Dey-Engley Neutralizing Broth ("D/E media") for a growth-no growth determination. That is, the D/E media was observed for turbidity after 48 hours at 37 °C. Turbidity being indicative of bacterial growth. The extent of continued growth on the treated microscope cover glasses was determined by viable plate counting using standard Nutrient Agar. The results of these analyses are provided in **Table II** and demonstrate that the diluted product solutions from Examples 10 and 11 kill >99.99% of the treated bacteria after 24 hours of contact.

**Table II**

| Sprayed Sample of | Log (CFU¹/ml) Reduction After | | | |
|---|---|---|---|---|
| | 10 min. | 1 hr. | 4 hr. | 24 hr. |
| Example 10 | 0 | 0 | 2 | 6 |
| Example 11 | 0 | 0 | 0 | 6 |

### Example 14

### Sanitization Efficacy of Silver Containing Films

Two sets of microscope cover glasses were pre-treated with silver containing films. Specifically, a film was sprayed on from the product solution of Example 10 (diluted to 90 ppm silver with deionized water) on one set of microscope cover glasses. A film was sprayed on from the product solution of Example 11 (diluted to 90 ppm silver with deionized water) on the other set of microscope cover glasses.

*Staphylococcus aureus* of ATCC 6538 strain was grown in a growth media (Nutrient Broth) and incubated at 37 °C. Two sets of pre-treated microscope cover glasses were inoculated with 10 µl of inoculum containing about 1x10⁶ bacteria per square inch of microscope cover glass. The microscope cover glasses were then subjected to multiple cycles of water rinsing, abrasion and re-inoculation. Microbial survival was determined as described in Example 21 after each wash cycle. In each case, efficacy of the treated samples was compared to a control population to account for die off due to the rinsing and abrasion procedures. Tests for which the control samples showed less than 10⁴ colonies per slide subsequent to rinsing and abrasion were considered invalid. The results are provided in **Table III** and demonstrate that the antimicrobial activity of films drawn from diluted product solutions from Examples 10 and 11 does not diminish after 4 successive rinse/abrasion cycles.

**Table III**

| Film drawn from product solution of | Log (CFU¹/ml) Reduction after | | | |
|---|---|---|---|---|
| | 1 cycle | 2 cycles | 3 cycles | 4 cycles |
| Example 10 | 6 | 6 | 6 | 6 |
| Example 11 | 6 | 6 | 6 | 6 |

### DISINFECTANT AND RESIDUAL EFFICACY DATA FOR ALCOHOL SOLUTIONS OF SILVER-POLYMER COMPLEX

### Methods and Test Description

Specific descriptions of the tests and summaries of the results are provided below for each section. For silver controls: AgNO₃ was diluted in ethanol/water 60/40 solution by weight at 100 ppm Ag (pH 6-6.5) and a second sample was also adjusted to pH 9 (with ammonia) for comparison to the high pH silver-polymer complexes.

The silver-polymer formulations and all silver nitrate controls used in contact kill or dry film residual efficacy evaluations were diluted to 100 ppm Ag in a solution of 60:40 ethanol:water by weight. The pH of the 60:40 ethanol/water was adjusted to 9 with ammonia (NH₃) when tested for comparison. Solutions of 60:40 ethanol/water served as controls for biological efficacy tests.

Glass slides were used for surface disinfection and residual efficacy studies. Formulations were tested at 100 ppm silver concentrations and 100 µl was applied to the glass surface before or after bacterial inoculation.

Bacteria used in the studies included *Pseudomonas aeruginosa* (ATCC 15442) and *Salmonella choleraesuis* (ATCC 10708). For surface studies, glass slides were inoculated with 10 µl of an overnight culture of bacteria at approximately 10⁶ colony forming units per milliliter (cfu/ml). Samples with bacteria were incubated at 37° C in a humidity cabinet. After the specific contact time, the slides were placed in DE neutralizing broth and surviving bacteria were enumerated using a most probable number method on trypticase soy broth (TSB) after 24 h hours at 37° C. Bacterial reduction was calculated versus the control (nontreated) samples taken at the specified time intervals.

The rinse residual efficacy studies were conducted by taking replicate slides at a specified time interval and rinsing in an up and down direction in deionized water for 15 seconds. The slides were blotted dry with sterile paper towels (3000 gram force on a balance) and re-inoculated. This cycle was repeated up to 5 times.

### Polymer-Silver Compositions Tested

The following silver-polymer formulations (Table IV) were used in the efficacy evaluation. Formulations contained various ratios of butyl acrylate (BA), 1-vinyl imidazole (VI), acrylic acid (AA), and lauryl acrylate (LA). Mixtures containing trimethylolpropane triacrylate (TMPTA) were cross-linked polymer systems. Samples without TMPTA were not cross-linked. All compositions were tested on an equal silver basis (final concentration = 100 ppm silver). The compositions made with TMPTA had an average particle size less than 10 nm.

**Table IV**

| **Sample #** | **Composition (%)** | **% Total Solids** | **% Silver** |
|---|---|---|---|
| 1 | 45BA/45VI/10AA | 11.75 | 1.35 |
| 2 | 40BA/40VI/10AA/10TMPTA | 10.89 | 1.25 |
| 3 | 40BA/40VI/10AA/10TMPTA | 23.20 | 2.45 |
| 4 | 45BA/45VI/10AA | 18.33 | 2.11 |
| 5 | 40LA/40VI/10AA/10TMPTA | 18.56 | 2.13 |
| 6 | 45LA/45VI/10AA | 16.99 | 1.96 |
| 7 | 40BA/40VI/10AA/10TMPTA | 17.81 | 2.04 |

### Example 15

### Contact Killing vs Bacteria on Surfaces (Disinfectant Testing)

Sterile glass slides were inoculated with bacterial inoculum (*Pseudomonas aeruginosa*) and dried for up to two hours. The formulations were then spread on the glass slides. Bacterial growth was evaluated after 30 seconds, and 1, 2, 5 and 10 minute contact times. All formulations were tested at 100 ppm Ag

The surface disinfection results showed that the five of the seven silver-polymer complexes provided equal killing (30 second efficacy) to silver nitrate alone at both low pH (6-6.5) and pH 9 (adjusted with ammonia, NH₃), indicating no loss of activity due to the polymer complex).

Both cross-linked and non-cross-linked polymer complexes were highly effective. All silver-polymer formulations demonstrated complete kill (less than detectable levels of bacteria) after the 1 minute contact time and showed improved activity compared to alcohol alone (2 minute efficacy). Results are provided in Table V.

**Table V**

| **Sample** | **Solution pH** | **Log Reduction at Specific Contact Times: (versus control)** | | | | |
|---|---|---|---|---|---|---|
| | | **30 Sec.** | **1 min** | **2 min** | **5 min** | **10 min** |
| 1 | 8.5 - 9 | 2.7 | > 4.4 | > 4,2 | > 4.0 | > 3.2 |
| 2 | 8.5 - 9 | > 3.7 | > 4.4 | > 4,2 | > 4.0 | > 3.2 |
| 3 | 8.5 - 9 | > 3.7 | > 4.4 | > 4,2 | > 4.0 | > 3.2 |
| 4 | 8.5 - 9 | > 3.7 | > 4.4 | > 4,2 | > 4.0 | > 3.2 |
| 5 | 8.5 - 9 | > 3.7 | > 4.4 | > 4,2 | > 4.0 | > 3.2 |
| 6 | 8.5 - 9 | > 3.7 | > 4.4 | > 4,2 | > 4.0 | > 3.2 |
| 7 | 8.5 - 9 | 2.7 | > 4.4 | > 4,2 | > 4.0 | > 3.2 |
| AgNO₃ | 6 - 6.5 | > 3.7 | > 4.4 | > 4,2 | > 4.0 | > 3.2 |
| AgNO₃ + NH₃ | 8.5 - 9 | > 3.7 | > 4.4 | > 4,2 | > 4.0 | > 3.2 |
| 60:40 ethanol:water | | 2.3 | 2.7 | > 4,2 | > 4.0 | > 3.2 |

### Example 16

### Hard Surface Residual Efficacy (Self-Sanitizing Efficacy)

The biocide formulation (tested at 100 ppm silver) was applied to glass slides, dried and inoculated (*Salmonella choleraesuis* and *Pseudomonas aeruginosa*). Bacterial growth was evaluated after 10 minutes, and 1, 4 and 24 hour contact times.

### All Formulations were tested at 100 ppm Ag

The residual efficacy studies showed that all seven silver-polymer complex (100 ppm silver) applied to a surface and allowed to dry, provided 1- to 4- hour killing (>3-log) versus bacteria when added to the treated surface. Most silver-polymer formulations showed significant kill within 1 hour against *Pseudomonas*, but required 4 hours for efficacy versus *Salmonella*. These tests also used the same alcohol:water diluent for testing and silver nitrate controls.

Results showed that the silver-polymer complex was similar to or more effective than silver nitrate alone (at pH 6-6.5 or adjusted to pH 9 with ammonia) indicating no loss of activity due to the polymer. Both cross-linked and non-cross-linked polymer complexes were highly effective. The ethanol-water control solution showed no residual efficacy after drying. Results are provided in Table VI.

**Table VI**

| **Sample** | **Log Reduction at Specific Contact Times (versus control)** | | | | | | | |
|---|---|---|---|---|---|---|---|---|
| | ***Pseudomonas aeruginosa*** | | | | ***Salmonella choleraesuis*** | | | |
| | 10 min | 1 hr | 4 hr | 24 hr | 10 min | 1 hr | 4 hr | 24 hr |
| 1 | 1.8 | > 6 | > 6 | > 6 | -0.5 | -0.7 | 4.1 | 4.1 |
| 2 | 0.8 | > 6 | > 6 | > 6 | -0.8 | -0.2 | > 4.2 | > 4.2 |
| 3 | 0.3 | 5.9 | 5.3 | > 6 | -0.8 | -0.4 | 4.1 | > 4.2 |
| 4 | 0.8 | 2.6 | > 6 | > 6 | -2.2 | 0.2 | > 4.2 | > 4.2 |
| 5 | 0.6 | 3.3 | > 6 | > 6 | -2.2 | -0.5 | > 4.2 | > 4.2 |
| 6 | 1.6 | 2.0 | > 6 | > 6 | -0.2 | -1.0 | > 4.2 | > 4.2 |
| 7 | 1.0 | 1.6 | 5.9 | > 6 | -0.4 | -0.8 | > 4.2 | > 4.2 |
| AgNO₃ | 1.0 | 3.6 | > 6 | > 6 | -1.0 | -0.5 | > 4.2 | > 4.2 |
| AgNO₃ + NH₃ | 1.0 | 2.6 | > 6 | > 6 | -0.2 | 0.0 | > 4.2 | > 4.2 |

### Example 17

### Multiple Wash Residual Efficacy (Self-Sanitizing Efficacy after Rinsing)

The formulation was applied on hard surface (sterile glass slides) and dried for 1-2 hours. The dry film was then washed up to a total of 5 times with agitation, dried with pressure and re-inoculated (with *S. choleraesuis*). Bacterial recovery was evaluated after 4 hour contact time.

Additional testing of the residual efficacy by washing the treated surfaces and re-inoculating with bacteria, showed that all seven silver-polymer complex (100 ppm silver) provided a minimum 3-log kill through 5 rinse-inoculation cycles. The silver nitrate adjusted to pH 9 with ammonia was less effective and failed to provide a 3-log kill after the 5^{th} rinse cycle. Ethanol-water alone failed after the first rinse (no efficacy with washing). This demonstrates persistent efficacy of the silver-polymer complex under added stress of water rinsing of a treated surface. Both cross-linked and non-cross-linked polymer complexes were highly effective. Results are provided in Table VII.

**Table VII**

| **Sample** | **Log Reduction after Specific Wash Cycles:** | | | | | |
|---|---|---|---|---|---|---|
| | **(4 hour contact time versus control)** | | | | | |
| **ID** | **0** | **1** | **2** | **3** | **4** | **5** |
| 1 | > 5.2 | > 5.0 | > 5.4 | 4.4 | 4.5 | 4.4 |
| 2 | > 5.2 | > 5.0 | > 5.4 | > 5.0 | > 5.2 | > 4.8 |
| 3 | > 5.2 | > 5.0 | > 5.4 | > 5.0 | > 5.2 | > 4.8 |
| 4 | > 5.2 | > 5.0 | > 5.4 | > 5.0 | > 5.2 | > 4.8 |
| 5 | > 5.2 | > 5.0 | > 5.4 | > 5.0 | 5.1 | 3.8 |
| 6 | > 5.2 | > 5.0 | > 5.4 | > 5.0 | > 5.2 | 4.4 |
| 7 | > 5.2 | > 5.0 | > 5.4 | > 5.0 | > 5.2 | > 4.8 |
| AgNO₃ | > 5.2 | > 5.0 | > 5.4 | > 5.0 | > 5.2 | > 4.8 |
| AgNO₃ + NH₃ | > 5.2 | > 5.0 | > 5.4 | 4.9 | 5.1 | 2.7 |
| ethanol + NH₃ | 0.8 | 0.4 | 0.7 | -0.2 | 0.2 | -0.2 |

### Example 18

### Multiple Wash and Wipe Residual Efficacy

The test was set up the same as the described above in C except that the slides were wiped with abrasion once after each wash cycle before inoculation.

Additional residual efficacy studies with a wiping-abrasion step, showed that 6 of 7 silver-polymer complex solutions (100 ppm silver) provided at least a 3-log kill efficacy through a minimum of 5 rinse-wiping-inoculation cycles. Both cross-linked and non-cross-linked polymer complexes were highly effective. Silver nitrate control samples (both pH values) failed to achieve a 3-log kill after 4 or 5 rinse-wipe cycles. Ethanol alone failed after the first rinse. This test demonstrates persistent efficacy of the silver-polymer complexes under added stress of manual wiping or abrasion and water rinsing of a treated surface and improved antimicrobial residual activity compared to AgNO₃ alone. Results are provided in Table VIII.

**Table VIII**

| **Sample** | **Log Reduction after Specific Wash Cycles:** | | | | | |
|---|---|---|---|---|---|---|
| | **(4 hour contact time versus control)** | | | | | |
| | **0** | **1** | **2** | **3** | **4** | **5** |
| 1 | > 5.0 | > 5.0 | > 4.8 | > 5.0 | 4.7 | 4.5 |
| 2 | > 5.0 | > 5.0 | 4.8 | > 5.0 | 4.9 | > 5.2 |
| 3 | > 5.0 | > 5.0 | 4.8 | > 5.0 | > 5.0 | 4.2 |
| 4 | > 5.0 | > 5.0 | > 4.8 | 4.9 | 4.9 | 5.1 |
| 5 | > 5.0 | > 5.0 | > 4.8 | > 5.0 | 4.9 | 4.5 |
| 6 | > 5.0 | > 5.0 | > 4.8 | > 5.0 | 4.9 | 3.5 |
| 7 | > 5.0 | 1.8 | 2.1 | 1.0 | 1.3 | 2.0 |
| AgNO₃ | > 5.0 | > 5.0 | > 4.8 | 4.9 | 4.6 | 1.0 |
| AgNO₃+NH₃ | > 5.0 | 4.9 | 4.4 | 3.3 | 1.0 | 1.0 |
| ethanol+NH₃ | 0.8 | 0.8 | -0.6 | 0.0 | 0.6 | 0.5 |

### Example 19

### Four Hour Wash Test (extended rinsing test)

The 100 ppm Ag diluted solution was spread on sterile glass slides and dried. The slides were then suspended in running water at 1200 ml/minute flow rate. After 4 hours of rinsing, the slides were air dried, inoculated (*Salmonella choleraesuis*) and evaluated for bacterial growth after 4 hour contact time. All Formulations were tested at 100 ppm Ag

A final test with the 100 ppm silver treated surface samples included a 4-hour water rinsing period followed by inoculation with bacteria. Results showed 6 of 7 silver-polymer complex samples still retained the 3-log killing efficacy after the extended rinse period. Some variation in polymer composition was observed. Both cross-linked and non-cross-linked polymer complexes were highly effective. Ethanol alone failed the rinsing test. The low pH silver nitrate (pH 6-6.5) samples showed excellent efficacy, however, the pH 9 samples adjusted with ammonia) showed no residual killing efficacy. Results are provided in Table IX

**Table IX**

| **Sample** | **Log Reduction after 4 hour contact time** | |
|---|---|---|
| | **(versus control)** | |
| | **No Wash** | **4 Hour Wash** |
| 1 | > 4.2 | > 4.2 |
| 2 | > 4.2 | 3.0 |
| 3 | > 4.2 | -0.5 |
| 4 | > 4.2 | 3.8 |
| 5 | > 4.2 | > 4.2 |
| 6 | > 4.2 | > 4.2 |
| 7 | > 4.2 | > 4.2 |
| AgNO₃ | > 4.2 | > 4.2 |
| AgNO₃ + NH₃ | > 4.2 | -0.2 |
| Alcohol + NH₃ | 0.5 | -0.8 |

## Claims

1. A method for sanitizing a surface; said method comprising applying to the surface an antimicrobial composition comprising:
(a) at least 50% of an alcohol selected from ethanol and isopropanol; and
(b) a metal complexed with a polymer, wherein the metal is selected from copper, silver, gold, tin, zinc and combinations thereof; and, wherein the polymer comprises monomer residues selected from residue A, residue B, residue C and mixtures thereof; with the proviso that the polymer contains no more than 99.5 wt% of monomer residues of residue B;
wherein residue A is 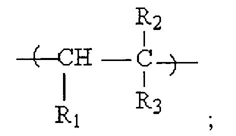 wherein residue B is 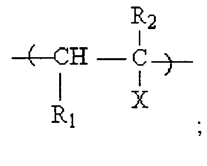 and
wherein residue C is 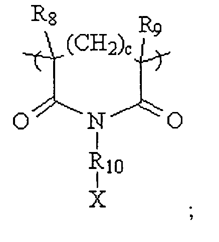 wherein
X is an unsaturated or aromatic heterocycle having at least one hetero atom selected from N, O and S;
c is 0 or 1;
R₁ is selected from H, CH₃ and -CO₂R₄; where R₄ is selected from H, CH₃, C₂H₅, a C₃-C₂₄ alkyl;
R₂ is selected from H, CH₃, C₂H₅, phenyl, -CH₂CO₂R₅ and -CO₂R₅; where R₅ is selected from (I)-(V),
(I) H;
(II) 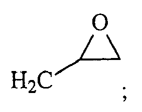
(III) -(CH₂CH(R₁₁)O)ₙH;
(IV) -(CH₂CH(R₁₁)O)ₙCOCH₂COCH₃; and,
(V) 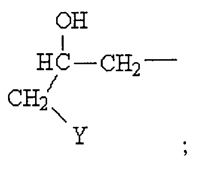
where R₁₁ is selected from H, methyl and phenyl; n is an integer from 1 to 20; Y is selected from OH, SO₃Z and X; where Z is selected from H, sodium, potassium and NH₄⁺; with the proviso that when the polymer contains 0 wt% of monomer residues of residue B and 0 wt% of monomer residues of residue C, R₂ is -CH₂CO₂R₅ or -CO₂R₅, R₅ is (V) and Y is X;
R₃ is selected from H, methyl, phenyl, sulfonated phenyl, phenol, acetate, hydroxy, a fragment O-R₁, where R₁ is as defined previously, -CO₂R₁₂ and -CONR₆R₇; where R₆ and R₇ are independently selected from H, methyl, ethyl, C(CH₃)₂CH₂SO₃Z, where Z is as defined previously, C₃-C₈ alkyl and a combined ring structure and R₁₂ is selected from H, CH₃, C₂H₅ and C₃-C₂₄ alkyl;
R₈ and R₉ are independently selected from hydrogen, methyl, ethyl and C₃-C₄ alkyl;
R₁₀ is selected from C₁-C₈ alkyl, C₂-C₈ alkenyl, C₆-C₁₀ unsaturated acyclic, C₆-C₁₀ cyclic, C₆-C₁₀ aromatic, C₂-C₄ alkylene oxide and poly (C₂-C₄ alkylene)_{b} oxides; where b is an integer from 2 to 20.

2. The method of claim 1, wherein the polymer further comprises at least 2 wt% cross-linker and wherein the polymer has an average particle size of less than 10 nm.

3. The method of claim 1, wherein the antimicrobial composition contains at least 50 ppm silver.

4. The method of claim 1, wherein the polymer comprises a copolymer of a heterocyclic containing monomer and a non-heterocyclic containing monomer.

5. The method of claim 4, wherein the ratio of the heterocyclic containing monomer to the non-heterocyclic containing monomer is between 95:5 to 5:95.

6. The method of claim 5, wherein the antimicrobial composition comprises a 1-vinylimidazole copolymer complexed with silver.

7. The method of claim 6, wherein the surface is a hard surface.

8. The method of claim 7, wherein the surface is sprayed with the antimicrobial composition.

9. The method of claim 6, wherein the surface is selected from among the group consisting of clay, wood, leather, fabric, rubber, paper and skin.

10. A method for sanitizing a surface; said method comprising applying to the surface an antimicrobial composition comprising:
(a) at least 50% of an alcohol selected from ethanol and isopropanol; and
(b) silver complexed with a polymer; wherein the polymer comprises at least 0.5 wt% crosslinker and at least 75 wt% of monomer residues selected from residue A, residue B, residue C and mixtures thereof;
wherein residue A is 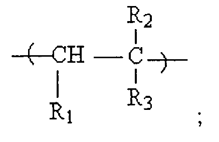 wherein residue B is 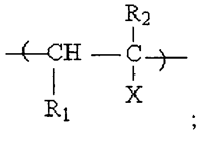 and
wherein residue C is 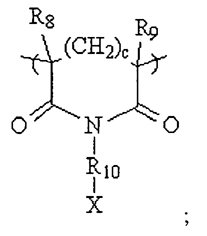 wherein
X is an unsaturated or aromatic heterocycle having at least one hetero atom selected from N, O and S;
c is 0 or 1;
R₁ is selected from H, CH₃ and -CO₂R₄; where R₄ is selected from H, CH₃, C₂H₅, a C₃-C₂₄ alkyl;
R₂ is selected from H, CH₃, C₂H₅, phenyl, -CH₂CO₂R₅ and -CO₂R₅; where R₅ is selected from (I)-(V),
(I) H;
(II) 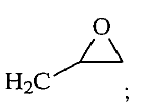
(III) -(CH₂CH(R₁₁)O)ₙH;
(IV) -(CH₂CH(R₁₁)O)ₙCOCH₂COCH₃; and,
(V) 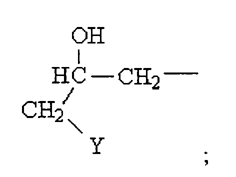
where R₁₁ is selected from H, methyl and phenyl; n is an integer from 1 to 20; Y is selected from OH, SO₃Z and X; where Z is selected from H, sodium, potassium and NH₄⁺; with the proviso that when the polymer contains 0 wt% of monomer residues of residue B and 0 wt% of monomer residues of residue C, R₂ is -CH₂CO₂R₅ or -CO₂R₅, R₅ is (V) and Y is X;
R₃ is selected from H, methyl, phenyl, sulfonated phenyl, phenol, acetate, hydroxy, a fragment O-R₁, where R₁ is as defined previously, -CO₂R₁₂ and -CONR₆R₇; where R₆ and R₇ are independently selected from H, methyl, ethyl, C(CH₃)₂CH₂SO₃Z, where Z is as defined previously, C₃-C₈ alkyl and a combined ring structure and R₁₂ is selected from H, CH₃, C₂H₅ and C₃-C₂₄ alkyl;
R₈ and R₉ are independently selected from hydrogen, methyl, ethyl and C₃-C₄ alkyl;
R₁₀ is selected from C₁-C₈ alkyl, C₂-C₈ alkenyl, C₆-C₁₀ unsaturated acyclic, C₆-C₁₀ cyclic, C₆-C₁₀ aromatic, C₂-C₄ alkylene oxide and poly (C₂-C₄ alkylene)_{b} oxides; where b is an integer from 2 to 20.
